# EUROPEAN PATENT APPLICATION

(11) **EP 4 233 678 A1**
(43) Date of publication of application: **30.08.2023**
(21) Application number: 23158692.6
(22) Date of filing: 27.02.2023
(51) Int. Cl.: A61B 1/00, A61B 1/005

(54) **DYNAMIC INPUT CONTROLS ON A DRIVE-BY-WIRE ENDOSCOPE**

(30) Priority: 28.02.2022 US 202217682792
(71) Applicant: Covidien LP, Mansfield, MA 02048 (US)
(72) Inventor: FRUSHOUR, Scott E.M., Boulder, 80301 (US)
(74) Representative: Maschio, Antonio

(57) **Abstract**

An endoscope including a proximal portion, a distal portion selectively articulatable in two planes of articulation, and a user interface disposed on a portion of the proximal portion, the user interface operably coupled to the distal portion such that inputs received on the user interface effectuate articulation of the distal portion, wherein a portion of the user interface is actuatable to inhibit articulation of the distal portion in one plane of articulation of the two planes of articulation via inputs on the user interface.

## Description

### BACKGROUND

### Technical Field

The present disclosure relates to the field of surgical devices, and in particular, to electronically controlled endoscopes.

### Description of Related Art

Minimally invasive surgical procedures have become increasingly prevalent over traditional open surgical techniques. Handheld endoscopes enable clinicians to explore internal cavities of a patient without the need for extensive incisions, reducing patient recovery time and patient discomfort. Endoscopes have been increasingly used to explore narrow luminal networks of the patient, such as the luminal network of the lungs, amongst others. Typical endoscopic procedures involve manual manipulation of the catheter and manual articulation of a distal end portion of the catheter to navigate the various bifurcations and/or undulating nature of the luminal network more easily.

Advances in technology has enabled clinicians to utilize motorized and/or robotic endoscopes to perform various surgical procedures. As can be appreciated, motorized or robotic systems enable fine motor control and a steadier grasp on the endoscope. However, clinicians have only recently begun training with these systems, and therefore, muscle memory and/or familiarity with the system is only in their infancy. As such, some clinicians may find it difficult to utilize the motorized functions of the endoscope, especially when trying to roll or otherwise twist the endoscope about its longitudinal axis. Clinicians have historically effectuated this rotation by flexion or extension of their wrist, a motion they have been using for years or decades. As such, utilizing motorized controls for this rotation during a surgical procedure may be cumbersome for clinicians who have not yet mastered the use of these endoscopes.

Further, as can be appreciated, navigation of the endoscope through the luminal network becomes more difficult the deeper into the luminal network the endoscope travels. As such, a clinician may wish to make large movements of the distal portion of the endoscope quickly at certain points in the luminal network, and may wish to make small, more precise movements of the distal portion of the endoscope at other points in the luminal network.

### SUMMARY

In accordance with the present disclosure, an endoscope includes a proximal portion, a distal portion selectively articulatable in two planes of articulation, and a user interface disposed on a portion of the proximal portion, the user interface operably coupled to the distal portion such that inputs received on the user interface effectuate articulation of the distal portion, wherein a portion of the user interface is actuatable to inhibit articulation of the distal portion in one plane of articulation of the two planes of articulation via inputs on the user interface.

In aspects, the user interface may include a joystick.

In certain aspects, the user interface may include a button pad.

In other aspects, the user interface may include an auxiliary switch, the auxiliary switch selectively actuatable to enable or disable articulation of the distal portion in one of the two planes of articulation.

In certain aspects, the endoscope may include a drive mechanism disposed within a portion of the proximal portion, the drive mechanism operably coupled to the user interface and operably coupled to the distal portion such that inputs received by the user interface cause the drive mechanism to effectuate articulation of the distal portion.

In other aspects, the user interface may be actuatable to inhibit actuation of a portion of the drive mechanism, such that the drive mechanism is constrained to articulating the distal portion in one plane of articulation of the two planes of articulation.

In aspects, a portion of the user interface may be actuatable to alter a scaling of inputs on the user interface to a speed of articulation of the distal portion.

In accordance with another aspect of the present disclosure, a system for performing a surgical procedure includes an endoscope including a distal portion selectively articulatable in two planes of articulation and a user interface operably coupled to the distal portion such that inputs received on the user interface effectuate articulation of the distal portion, and a workstation operably coupled to the endoscope, the workstation including a memory and a processor, the memory storing instructions, which when executed by the processor cause the processor to receiving an input and in response to the input, inhibit articulation of the distal portion of the endoscope in one plane of articulation of the two planes of articulation.

In aspects, the instructions, when executed by the processor, may alter a scaling of inputs on the user interface to a speed of articulation of the distal portion in response to the input.

In certain aspects, the endoscope may include a drive mechanism operably coupled to the user interface and operably coupled to the distal portion such that inputs received by the user interface cause the drive mechanism to effectuate articulation of the distal portion.

In other aspects, receiving an input may include receiving an input from an auxiliary switch operably coupled to the user interface.

In certain aspects, receiving an input may include receiving an input from the instructions stored on the memory.

In aspects, the user interface may include a joystick.

In accordance with another aspect of the present disclosure, a method of performing a surgical procedure includes receiving an input to inhibit articulation of a distal portion of an endoscope in one plane of articulation of two planes of articulation, receiving an input to articulate the distal portion of the endoscope in the one plane of articulation, and manipulating the endoscope to rotate the distal portion of the endoscope to a desired direction.

In aspects, receiving the input may include receiving an input on a user interface operably coupled to the distal portion of the endoscope.

In other aspects, the method may include receiving a second input to alter a scaling of inputs on the user interface to a speed of articulation of the distal portion.

In certain aspects, receiving the input may include actuating an auxiliary button disposed on a portion of the endoscope to inhibit articulation of the distal portion of the endoscope in one plane of articulation of the two planes of articulation.

In other aspects, receiving the input may include receiving an input from a workstation operably coupled to the endoscope.

In aspects, manipulating the endoscope may include rotating the endoscope in a first direction to rotate the distal portion of the endoscope to the desired direction.

In certain aspects, the method may include causing a drive mechanism to articulate the distal portion of the endoscope in the one plane of articulation in response to the input.

### BRIEF DESCRIPTION OF THE DRAWINGS

Various aspects and embodiments of the disclosure are described hereinbelow with references to the drawings, wherein:
FIG. 1 is a schematic view of a surgical system provided in accordance with the present disclosure;
FIG. 2 is an exploded view of a drive mechanism of an endoscope of the surgical system of FIG. 1;
FIG. 3 is a perspective view of a proximal portion of the endoscope of FIG. 2;
FIG. 4 is a perspective view of a distal portion of the endoscope of FIG. 2;
FIG. 5 is a perspective view of proximal portion of another embodiment of the endoscope of FIG. 2;
FIG. 6 is a flow diagram of a method of performing a surgical procedure provided in accordance with the present disclosure;
FIG. 7 is a perspective view of a robotic surgical system of the surgical system of FIG. 1; and
FIG. 8 is a schematic view of a workstation of the surgical system of FIG. 1.

### DETAILED DESCRIPTION

The present disclosure is directed to a surgical system having an endoscope operably coupled to a workstation. The endoscope includes a drive mechanism disposed within a portion thereof that is configured to effectuate articulation of a distal portion of the endoscope in two planes of articulation. The endoscope includes a user interface that is configured to translate inputs received by the user interface into a corresponding articulation of the distal portion of the endoscope. The user interface may include an auxiliary button or switch that is configured to enable or disable articulation of the distal portion in one of the two planes of articulation. In this manner, the user interface is utilized to articulate or otherwise curve the distal portion to a desired shape. At this point, the user may rotate the endoscope by flexion or extension of their wrist to rotate the distal portion of the endoscope to the desired location. As can be appreciated, by restricting articulation of the distal portion of the endoscope to one plane of articulation, clinicians who may not be as familiar or comfortable utilizing an endoscope with two plane articulation may more effectively use the endoscope during the surgical procedure.

It is envisioned that articulation of the distal portion may be constrained by the auxiliary switch or via the workstation. In this manner, the distal portion may be constrained to one plane of articulation before the surgical procedure begins and may act as a safeguard against accidental enabling of two plane articulation during the surgical procedure.

It is envisioned that the speed at which the distal portion of the endoscope is articulated may be altered by scaling the inputs received on the user interface. In this manner, if it is desired to more quickly articulate the distal portion of the endoscope, the inputs to the user interface may be scaled up. In contrast, if more control is desired and therefore, a slower speed is desired, the inputs to the user interface may be scaled down. It is contemplated that the scaling may be effectuated by toggling the auxiliary switch or may be effectuated by using the workstation. In embodiments, the scaling may be automatically adjusting depending upon the sensed location of the distal portion of the endoscope within a luminal network of the body. In this manner, if the workstation senses that the distal portion of the endoscope is in larger airways with few bifurcations, the scaling may be increased in order to permit the clinician to more quickly adjust the articulation of the distal portion. Similarly, if the workstation senses that the distal portion of the endoscope is in smaller airways with many bifurcations, the scaling may be decreased in order to enable the clinician to have finer control over the articulation of the distal portion of the endoscope. These and other aspects of the present disclosure are described in further detail herein.

Although generally described with reference to the lung, it is contemplated that the systems and method described herein may be used with any structure within the patient's body, such as the liver, kidney, prostate, gynecological, amongst others.

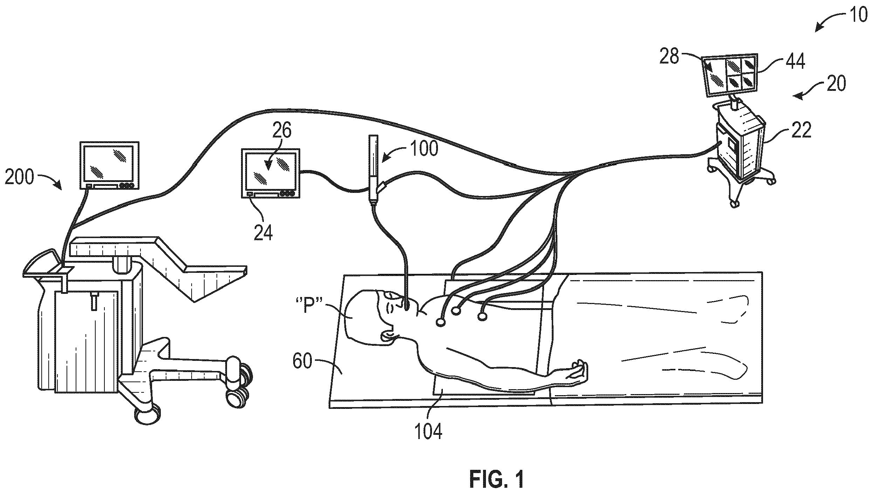

Turning now to the drawings, FIG. 1 surgical system provided in accordance with the present disclosure and generally identified by reference numeral 10. As will be described in further detail hereinbelow, the surgical system 10 is generally configured to identify target tissue, register real-time images captured by a surgical instrument to a generated 3-Dimensional (3D) model, and navigate the surgical instrument to the target tissue.

The surgical system includes an endoscope 100, a workstation 20 operably coupled to the endoscope 100, and a robotic surgical system 200 operably coupled to the workstation 20 and operably coupled to the endoscope 100. The patient "P" is shown lying on an operating table 60 with the endoscope 100 inserted through the patient's mouth and into the patient's airways, although it is contemplated that the endoscope 100 may be inserted into any suitable body cavity of the patient, depending upon the procedure being performed.

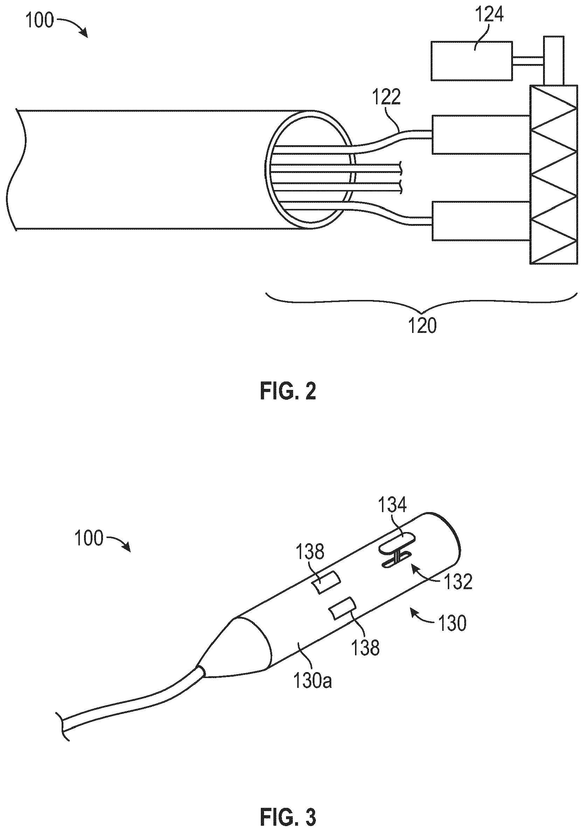

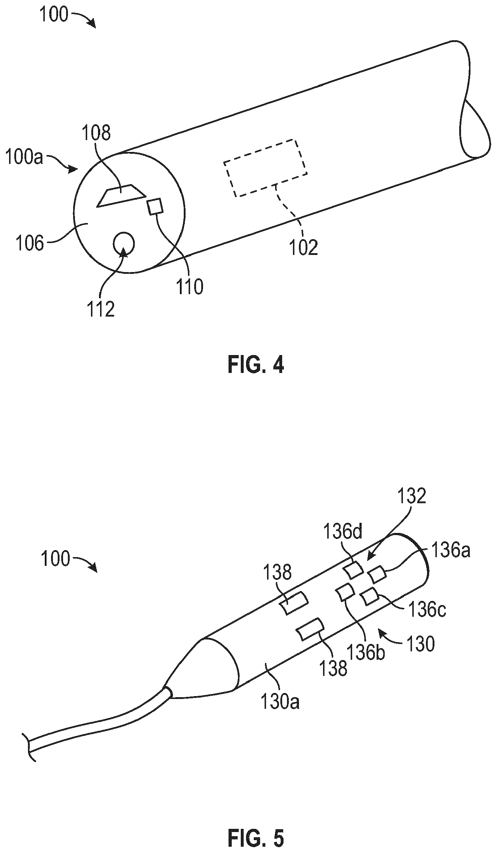

With reference to FIG. 2, the endoscope 100 includes a drive mechanism 120 disposed within an interior portion thereof that is operably coupled to a proximal portion of the endoscope 100, although it is envisioned that the drive mechanism 120 may be operably coupled to any portion of the endoscope 100. The drive mechanism 120 effectuates manipulation or articulation of a distal portion 100a (FIG. 4) of the endoscope 100 in four degrees of freedom or two planes or articulation (e.g., left, right, up, down), which is controlled by two push-pull wires, although it is contemplated that the drive mechanism 120 may include any suitable number of wires to effectuate movement and/or articulation of the distal portion 100a of the endoscope 100 in greater or fewer degrees of freedom without departing from the scope of the present disclosure. It is contemplated that the distal portion 100a of the endoscope 100 may be manipulated in more than two planes of articulation, such as in polar coordinates, or may maintain an angle of the distal portion 100a relative to the longitudinal axis of the endoscope while altering the azimuth of the distal portion 100a, or vice versa. In one non-limiting embodiment, the system 10 may define a vector or trajectory of the distal portion 100a of the endoscope in relation to the two planes of articulation.

It is envisioned that the drive mechanism 120 may be cable actuated using artificial tendons or pull wires 122 (*e.g.,* metallic, non-metallic, composite, etc.) or may be a nitinol wire mechanism. In embodiments, the drive mechanism 120 may include motors 124 or other suitable devices capable of effectuating movement of the pull wires 122. In this manner, the motors 124 are disposed within the endoscope 100 such that rotation of the motors 124 effectuates a corresponding articulation of the distal portion 100a of the endoscope 100.

Turning to FIGS. 3-5, the endoscope 100 includes a control handle 130 disposed at a proximal portion thereof that is configured to be grasped by a user. The control handle 130 is operably coupled to the endoscope 100 such that rotation and/or manipulation of the control handle 130 effectuates a corresponding rotation of the distal portion 100a of the endoscope 100. The control handle 130 includes a user interface 132 disposed on an outer portion thereof. The user interface 132 includes a joystick 134 protruding from an outer surface 130a of the control handle 130 that is configured to be manipulated by a user. In embodiments, the joystick 134 may be manipulated by a user's thumb and may be manipulated in two axes (e.g., forward-backward and left-right), although it is contemplated that the joystick 134 may be configured to be manipulated using any suitable means and may be manipulated in any number of axes, and in embodiments, may be manipulated 360-degrees about an axis. As can be appreciated, the joystick 134 may be utilized by the user to manipulate the distal portion 100a of the endoscope 100 in two planes of articulation, such as left, right, up, down, and in embodiments, may be utilized to rotate the distal portion 100a of the endoscope 100 clockwise or counterclockwise about a longitudinal axis of the endoscope 100. Although generally described as being a joystick, it is envisioned that the user interface may utilize a scroll wheel, toggle switch, slider (analog, digital, capacitive touch, etc.), trackball, or other suitable digital and electromechanical interfaces.

It is contemplated that in lieu of a joystick 134, the user interface 132 may include one or more buttons 136a-d (FIG. 5) disposed on the outer surface 130a of the control handle 130. In embodiments, the one or more buttons 136a-d may be in a configuration of a four button pad, having a first button 136a, a second button 136b, a third button 136c, and a fourth button 136d disposed in a cross or plus-sign configuration such that the first and second buttons 136a, 136b are longitudinally aligned on the endoscope 100 and the third and fourth buttons 136c, 136d are aligned in a transverse direction to the first and second buttons 136a, 136b. Although generally described as being four separate buttons, it is contemplated that the four-button pad may be a rocker pad or other suitable type of input device without departing from the scope of the present disclosure. As can be appreciated, the first and second buttons 136a, 136b correspond to up and down motions whereas the third and fourth buttons 136c, 136d correspond to left and right motions. In embodiments, the user interface may include a return to neutral or return to center switch or button (not shown) to quickly return the orientation of the distal portion 100a of the endoscope 100 to center (e.g., unarticulated) should the user become disoriented or need to quickly reset the position of the distal portion 100a. It is envisioned that the return to center button may be an analog button, a digital button, a capacitive touch button, etc. In embodiments, the return to neutral function may be actuated using any of the user interface buttons, such as the joystick 134, the one or more buttons 136a-d, the auxiliary switch 138 (described hereinbelow) using a gesture or other function, such as double-tapping the buttons or switches or depressing two or more switches or buttons simultaneously.

Continuing with FIGS. 3-5, it is envisioned that the user interface 132 may include one or more auxiliary switches or buttons 138 disposed on a portion of the outer surface 130a of the control handle 130 that are selectively actuatable by the user. In embodiments, the auxiliary buttons 138 may be utilized to enable or disable software features stored on the workstation 20, such as increasing or decreasing a scale associated with the inputs to the joystick 134 and/or buttons 136a-d or enabling or disabling manipulation of the distal portion 100a of the endoscope 100 in one plane of articulation to enable a user to control roll of the endoscope 100 in a more traditional, manual fashion. Although generally described as being a digital or electronic lockout, it is envisioned that the one or more auxiliary switches 138 may mechanically lock out articulation of the distal portion 100a in one plane using any suitable means. In embodiments, the auxiliary switch 138 may selectively lock or otherwise reduce mobility of the distal portion 100a. In this manner, the auxiliary switch 138 may be utilized to momentarily reduce the scaling or sensitivity of the user interface (e.g., the joystick 134, the one or more buttons 136a-d, etc.) when the user needs to perform a critical or otherwise delicate articulation of the distal portion 100a.

As can be appreciated, clinicians have only recently begun training with motorized endoscopes, and therefore, muscle memory and/or familiarity with the system is only in its infancy. As such, some clinicians may find it difficult to utilize the motorized functions of the endoscope in two planes of articulation, especially when trying to roll or otherwise twist the endoscope about its longitudinal axis. Clinicians have historically effectuated this rotation by flexion or extension of their wrist, a motion they have been using for years or decades. Therefore, by lockout out or otherwise disabling articulation of the distal portion 100a of the endoscope 100 in one plane of articulation, the clinician is able to utilize the motorized control to effectuate articulation of the distal portion 100a in one plane, while utilizing flexion and extension of their wrist to effectuate roll of the endoscope 100 in a more traditional manner.

During navigation of the distal portion 100a of the endoscope 100 through the luminal network, the precision and care required to successfully and carefully navigate the distal portion 100a through the lumens varies. As can be appreciated, initial navigation of the distal portion 100a of the endoscope 100 is through lumens having a relatively large diameter and fewer bifurcations as compared to later stages of navigation. As such, a user is able to more quickly advance and articulate the distal portion 100a of the endoscope 100 within the luminal network without impacting or otherwise abutting the tissue walls. Navigation of the distal portion 100a of the endoscope 100 becomes more difficult as the lumens become increasingly narrower and the number of bifurcations increases, requiring finer and slower articulation of the distal portion 100a of the endoscope 100.

With a manual endoscope, the clinician would increase or decrease the speed using hand movements, increasing or decreasing the speed at which the distal end portion 100a of the endoscope 100 is advanced and rotated within the luminal network by moving one's hands faster or slower. As can be appreciated, electronic control of articulation of the distal end portion 100a of the endoscope 100, such as employing the drive mechanism 120 and user interface 132, often utilizes a single speed or in the case of a joystick, a proportional speed control. However, the proportional control is set at a rate that may be either too slow or too fast, depending upon the location at which the distal end portion 100a of the endoscope 100 is being navigated.

The present disclosure enables the user to increase or decrease the speed at which the distal end portion 100a of the endoscope 100 is articulated by scaling the input up or down. In this manner, if the clinician wishes to increase or decrease the rate at which the distal end portion 100a of the endoscope 100 is articulated or wishes to have finer control over articulation of the distal end portion 100a, the user can toggle or otherwise engage the auxiliary switch 138 to increase or decrease the scaling of the input to the joystick 134 or buttons 136a-d, as is appropriate. It is contemplated that the scaling of inputs may be applied equally to both planes of articulation, applied to one plane of articulation, split amongst both planes of articulation (*e.g*., one plane scaled differently than the other, etc. It is envisioned that the scaling may be ratiometric, such that if the scaling of the first plane of articulation is altered, the scaling of the second plane of articulation is likewise altered by the same fractional scale. Although generally described as being the same ratio, it is envisioned that the first plane of articulation and the second plane of articulation may be scaled at a differing rate or ratio, such as two to one, one half, three to one, etc.

Additionally, the user interface 132 enables a user to enable or disable articulation of the distal portion 100a of the endoscope 100 in one of the two planes of articulation to mimic a more traditional, manual endoscope. In this manner, the user may toggle or otherwise engage the auxiliary switch 138 to enable two-plane articulation of the distal portion 100a of the endoscope 100 or disable two-plane articulation of the distal portion 100a and restrict articulation of the distal portion 100a to a single plane of articulation. As can be appreciated, restricting articulation of the distal portion 100a of the endoscope 100 to a single plane of articulation enables a clinician to roll the endoscope 100 in the desired direction (*e.g.,* clockwise or counterclockwise) by using flexion or extension of their wrist to effectuate the desired rotation of the endoscope 100.

Although generally described as being manually selected during the surgical procedure, it is envisioned that the scaling of inputs to the user interface 132 and restricting articulation of the distal portion 100a of the endoscope 100 to a single plane of articulation may be effectuated by use of the workstation 20 either before beginning or during the surgical procedure. As can be appreciated, setting the scaling and type of articulation using the workstation 20 may help avoid changing these settings by the user inadvertently depressing or actuating the auxiliary switch 138. In embodiments, the user interface 132 may require multiple buttons or auxiliary switches to be depressed simultaneously to effectuate a change in the scaling or number of planes of articulation. It is envisioned that the workstation 20 may automatically adjust the scaling depending upon the sensed location of the distal portion 100a of the endoscope within the luminal network. In this manner, the workstation 20 may automatically select the appropriate scaling corresponding to the location of the distal portion 100a within the luminal network, or a user may specify one or more scaling's to be utilized at certain locations within the luminal network.

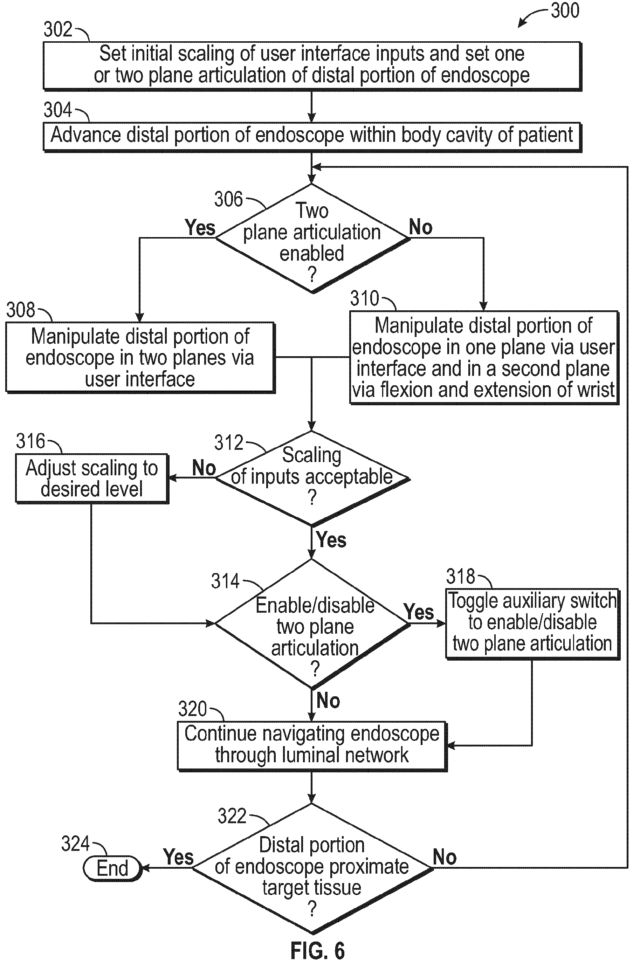

With reference to FIG 6, a method of performing a surgical procedure is illustrated and generally identified by reference numeral 300. Initially, at step 302, the endoscope is placed in the desired operative state, setting the initial scaling of the user interface 132 inputs and/or enabling or disabling two plane articulation of the distal portion 100a of the endoscope 100. Thereafter, at step 304, the distal portion 100a of the endoscope 100 is advanced into a body cavity of a patient. With the distal portion 100a located within a body cavity of the patient, at step 306, a determination is made as to if two plane articulation of the distal portion 100a is enable or disabled. If two plane articulation of the distal portion 100a is enabled, the distal portion 100a of the endoscope 100 is manipulated in two planes using the user interface 132 at step 308. If two plane articulation of the distal portion 100a is disabled, the distal portion 100a of the endoscope is articulated in one plane using the user interface 132 and the endoscope is rotated by flexion or extension of the user's wrist at step 310. With the distal portion 100a of the endoscope 100 in the desired orientation, at step 312, a determination is made as to if the scaling of the inputs on the user interface 132 is acceptable. If so, at step 314, a determination is made as to whether two plane articulation of the distal portion 100a should be enabled or disabled. If the scaling is not acceptable, at step 316, the scaling is adjusted to the desired level and a determination of whether to enable or disable two plane articulation of the distal portion 100a is made at step 314. If a change in the status of two plane articulation of the distal portion 100a is needed, the auxiliary switch 318 is toggled to enable or disable two plane articulation and navigation of the endoscope 100 through the luminal network is continued at step 320. If no change in the status of two plane articulation of the distal portion 100a is needed, navigation of the endoscope 100 through the luminal network is continued at step 320. During continued navigation of the endoscope 100 through the luminal network of the patient, a determination is made at step 322 as to if the distal portion 100a of the endoscope 100 is proximate the target tissue. If so, the process ends at step 324. Otherwise, the process returns to step 306. As can be appreciated, the method 300 may be repeated as many times as necessary until the distal portion 100a of the endoscope 100 is located proximate the target tissue.

Returning to FIG. 4, it is contemplated that the endoscope 100 may include a distal surface 106 having a camera 108 disposed thereon. Although generally illustrated as having one camera 108, it is contemplated that the endoscope 100 may include any number of cameras disposed on the distal surface 106 or any other suitable location thereon (*e.g.,* a sidewall, etc.). It is envisioned that the camera 108 may be a complementary metal-oxide-semiconductor (CMOS) camera, and in embodiments, may be a mini-CMOS camera. As can be appreciated, the camera 108 captures images of the patient's anatomy from a perspective of looking out from the distal surface 106. Although generally identified as being a CMOS camera, it is envisioned that the camera 108 may be any suitable camera, such as a charge-coupled device (CCD), N-type metal-oxide-semiconductor (NMOS), and in embodiments, may be a white light camera, infrared (IR) camera, Narrow Band Imaging (NBI) camera, various filters, amongst others, depending upon the design needs of the system 10. In embodiments, the endoscope may include an ultrasound transducer and receiver, microscopy, Optical Coherence Tomography (OCT), spectroscopy, or other suitable modalities capable of imaging the patient's anatomy.

In embodiments, the endoscope 100 may include one or more light sources 110 disposed on the distal surface 106 or any other suitable location (e.g., aside surface, a protuberance, etc.). The light source 110 may be or may include a light emitting diode (LED), an optical fiber connected to a light source that is located external to the patient, amongst others, and may emit white, IR, or near infrared (NIR) light. In this manner, the camera 108 may be a white light camera, IR camera, or NIR camera, a camera that is capable of capturing white light and NIR light, amongst others. In one non-limiting embodiment, the camera 108 is a white light mini-CMOS camera.

The endoscope 100 includes one or more working channels 112 defined therethrough and extending through the distal surface 106. The working channel 112 is configured to receive a tool (not shown), locatable guide (LG), amongst others to enable a clinician to navigate to, or treat, target tissue. It is contemplated that the tool may be any suitable tool utilized during an endoscopic surgical procedure, and in embodiments, may be a fixed tool.

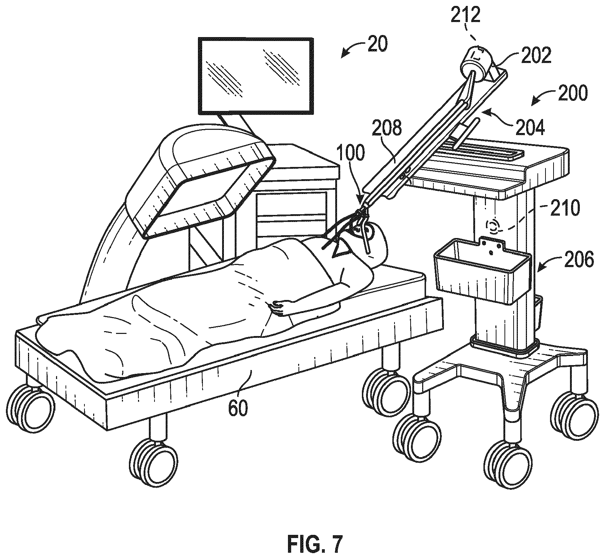

With reference to FIG. 7, the robotic surgical system 200 includes a drive mechanism 202 including a robotic arm 204 operably coupled to a base or cart 206. The robotic arm 204 includes a cradle 208 that is configured to receive a portion of the endoscope 100 thereon. The endoscope 100 is coupled to the cradle 208 using any suitable means (*e.g.,* straps, mechanical fasteners, couplings, amongst others). It is envisioned that the robotic surgical system 200 may communicate with the endoscope 100 via electrical connection (*e.g.,* contacts, plugs, etc.) or may be in wireless communication with the endoscope 100 to control or otherwise effectuate movement of the motors 124 and receive images captured by the camera 108. In this manner, it is contemplated that the robotic surgical system 200 may include a wireless communication system 210 operably coupled thereto such that the endoscope 100 may wirelessly communicate with the robotic surgical system 200 and/or the workstation 20 via Wi-Fi, Bluetooth°, amongst others. As can be appreciated, the robotic surgical system 200 may omit the electrical contacts altogether and may communicate with the endoscope 100 wirelessly or may utilize both electrical contacts and wireless communication. The wireless communication system 210 is substantially similar to the wireless network interface 28 of the workstation 20, and therefore, the wireless communication system 210 will not be described in detail herein in the interest of brevity. In embodiments, the robotic surgical system 200 and the workstation 20 may be one in the same or may be widely distributed over multiple locations within the operating room. It is contemplated that the workstation 20 may be disposed in a separate location and the display 12 may be an overhead monitor disposed within the operating room.

Although generally described as having the motors 124 disposed within the endoscope 100, it is contemplated that the endoscope 100 may not include motors 124 disposed therein. In this manner, the drive mechanism 120 disposed within the endoscope 100 may interface with motors 124 disposed within the cradle 208 of the robotic surgical system 200. In embodiments, the endoscope 100 may include a motor or motors 124 for controlling articulation of the distal portion 100a of the endoscope 100 in one plane (*e.g.,* left/null, right/null, etc.) and the drive mechanism 202 of the robotic surgical system 200 may include at least one motor 212 to effectuate the second axis of rotation and for axial motion. In this manner, the motor 124 of the endoscope 100 and the motors 212 of the robotic surgical system 200 cooperate to effectuate four-way articulation of the distal portion 100a of the endoscope 100 and effectuate rotation of the endoscope 100. As can be appreciated, by removing the motors 124 from the endoscope 100, the endoscope 100 becomes increasingly cheaper to manufacture and may be a disposable unit. In embodiments, the endoscope 100 may be integrated into the robotic surgical system 200 (*e.g.,* one piece) and may not be a separate component.

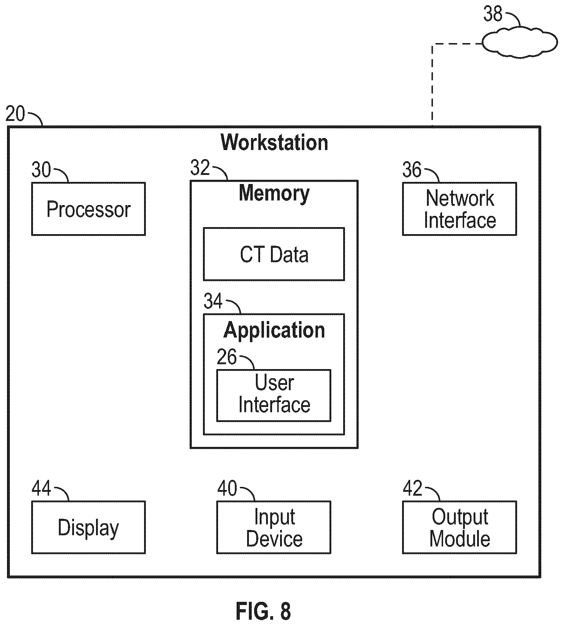

Returning to FIG. 1 and with additional reference to FIG. 8, the workstation 20 includes a computer 22 and a display 24 that is configured to display one or more user interfaces 26 and/or 28. The workstation 20 may be a desktop computer or a tower configuration with the display 24 or may be a laptop computer or other computing device. The workstation 20 includes a processor 30 which executes software stored in a memory 32. The memory 32 may store video or other imaging data captured by the endoscope 100 or pre-procedure images from, for example, a computer-tomography (CT) scan, Positron Emission Tomography (PET), Magnetic Resonance Imaging (MRI), Cone-beam CT, amongst others. In addition, the memory 32 may store one or more applications 34 to be executed by the processor 30. Though not explicitly illustrated, the display 24 may be incorporated into a head mounted display such as an augmented reality (AR) headset such as the HoloLens offered by Microsoft Corp.

A network interface 36 enables the workstation 20 to communicate with a variety of other devices and systems via the Internet. The network interface 36 may connect the workstation 20 to the Internet via a wired or wireless connection. Additionally, or alternatively, the communication may be via an ad-hoc Bluetooth° or wireless network enabling communication with a wide-area network (WAN) and/or a local area network (LAN). The network interface 36 may connect to the Internet via one or more gateways, routers, and network address translation (NAT) devices. The network interface 36 may communicate with a cloud storage system 38, in which further image data and videos may be stored. The cloud storage system 38 may be remote from or on the premises of the hospital such as in a control or hospital information technology room. An input module 40 receives inputs from an input device such as a keyboard, a mouse, voice commands, amongst others. An output module 42 connects the processor 30 and the memory 32 to a variety of output devices such as the display 24. In embodiments, the workstation 20 may include its own display 44, which may be a touchscreen display.

In embodiments, the endoscope 100 includes a location sensor, such as an electromagnetic (EM) sensor 102 (FIG. 4) which receives electromagnetic signals from an electromagnetic field generator 104 (FIG. 1) which generates one or more electromagnetic fields. In one non-limiting embodiment, the EM field generator 104 generates three or more electromagnetic fields. It is envisioned that the EM sensor 102 may be a single coil sensor that enables the system 10 to identify the position of the endoscope 100 within the EM field generated by the EM field generator 104, although it is contemplated that the EM sensor 102 maybe any suitable sensor and may be a sensor capable of enabling the system 10 to identify the position, orientation, and/or pose of the endoscope 100 within the EM field.

With continued reference to FIG. 8, one of the applications 34 stored in the memory 32 and executed by the processor 30 may determine the position of the EM sensor 102 in the EM field generated by the electromagnetic field generator 104. The determination of the position of the endoscope 100 and one or more cameras disposed thereon enables one method in which the images captured by the endoscope may be registered to a generated 3D model of the patient's anatomy, as will be described in further detail hereinbelow. Although generally described as being an EM sensor, it is contemplated that other position sensors may be utilized, such as an ultrasound sensor, flex sensors, fiber Bragg grating (FBG), robotic position detection sensors (*e.g.,* motor displacement sensors, pose sensors, etc.), amongst others.

In a planning or pre-procedure phase, the software stored in the memory 32 and executed by the processor 30 utilizes pre-procedure CT image data, either stored in the memory 32 or retrieved via the network interface 36, for generating a viewing a 3D model of the patient's anatomy, enabling the identification of target tissue on the 3D model (automatically, semiautomatically), or manually, and in embodiments, allowing for the selection of a pathway through the patient's anatomy to the target tissue. One example of such an application is the ILOGIC° planning and navigation suites currently marketed by Medtronic. The 3D model may be displayed on the display 24 or another suitable display (not shown) associated with the workstation 20, or in ay other suitable fashion. Using the workstation 20, various views of the 3D model may be provided and/or the 3D model may be manipulated to facilitate identification of the target tissue on the 3D model and/or selection of a suitable pathway to the target tissue.

Although generally described hereinabove, it is envisioned that the memory 32 may include any non-transitory computer-readable storage media for storing data and/or software including instructions that are executable by the processor 30 and which control the operation of workstation 20 and, in some embodiments, may also control the operation of the endoscope 100. In an embodiment, memory 32 may include one or more storage devices such as solid-state storage devices, e.g., flash memory chips. Alternatively, or in addition to the one or more solid-state storage-devices, the memory 32 may include one or more mass storage devices connected to the processor 30 through a mass storage controller (not shown) and a communications bus (not shown).

Although the description of computer-readable media contained herein refers to solid-state storage, it should be appreciated by those skilled in the art that computer-readable storage media can be any available media that can be accessed by the processor 30. That is, computer readable storage media may include non-transitory, volatile and non-volatile, removable and non-removable media implemented in any method or technology for storage of information such as computer-readable instructions, data structures, program modules, or other data. For example, computer-readable storage media may include RAM, ROM, EPROM, EEPROM, flash memory or other solid-state memory technology, CD-ROM, DVD, Blu-Ray or other optical storage, magnetic cassettes, magnetic tape, magnetic disk storage or other magnetic storage devices, or any other medium which may be used to store the desired information, and which may be accessed by the workstation 20.

While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplification of embodiments. Those skilled in the art will envision other modifications within the scope and spirit of the claims appended hereto.

The invention may be described by reference to the following numbered paragraphs:-
1. An endoscope, comprising:
   a proximal portion;
   a distal portion selectively articulatable in two planes of articulation; and
   a user interface disposed on a portion of the proximal portion, the user interface operably coupled to the distal portion such that inputs received on the user interface effectuate articulation of the distal portion,
   wherein a portion of the user interface is actuatable to inhibit articulation of the distal portion in one plane of articulation of the two planes of articulation via inputs on the user interface.
2. The endoscope according to paragraph 1, wherein the user interface includes a joystick.
3. The endoscope according to paragraph 1, wherein the user interface includes a button pad.
4. The endoscope according to paragraph 1, wherein the user interface includes an auxiliary switch, the auxiliary switch selectively actuatable to enable or disable articulation of the distal portion in one of the two planes of articulation.
5. The endoscope according to paragraph 1, further comprising a drive mechanism disposed within a portion of the proximal portion, the drive mechanism operably coupled to the user interface and operably coupled to the distal portion such that inputs received by the user interface cause the drive mechanism to effectuate articulation of the distal portion.
6. The endoscope according to paragraph 5, wherein the user interface is actuatable to inhibit actuation of a portion of the drive mechanism, such that the drive mechanism is constrained to articulating the distal portion in one plane of articulation of the two planes of articulation.
7. The endoscope according to paragraph 1, wherein a portion of the user interface is actuatable to alter a scaling of inputs on the user interface to a speed of articulation of the distal portion.
8. A system for performing a surgical procedure, comprising:
   an endoscope, the endoscope including:
      a distal portion selectively articulatable in two planes of articulation; and
      a user interface, the user interface operably coupled to the distal portion such that inputs received on the user interface effectuate articulation of the distal portion; and
   a workstation operably coupled to the endoscope, the workstation including a memory and a processor, the memory storing instructions, which when executed by the processor cause the processor to:
      receive an input; and
      in response to the input, inhibit articulation of the distal portion of the endoscope in one plane of articulation of the two planes of articulation.
9. The system according to paragraph 8, wherein the instructions, when executed by the processor, altering a scaling of inputs on the user interface to a speed of articulation of the distal portion in response to the input.
10. The system according to paragraph 8, wherein the endoscope further includes a drive mechanism, the drive mechanism operably coupled to the user interface and operably coupled to the distal portion such that inputs received by the user interface cause the drive mechanism to effectuate articulation of the distal portion.
11. The system according to paragraph 8, wherein receiving an input includes receiving an input from an auxiliary switch operably coupled to the user interface.
12. The system according to paragraph 8, wherein receiving an input includes receiving an input from the instructions stored on the memory.
13. The system according to paragraph 8, wherein the user interface includes a joystick.
14. A method of performing a surgical procedure, comprising:
   receiving an input to inhibit articulation of a distal portion of an endoscope in one plane of articulation of two planes of articulation;
   receiving an input to articulate the distal portion of the endoscope in the one plane of articulation; and
   manipulating the endoscope to rotate the distal portion of the endoscope to a desired direction.
15. The method according to paragraph 14, wherein receiving the input includes receiving an input on a user interface operably coupled to the distal portion of the endoscope.
16. The method according to paragraph 14, further comprising receiving a second input to alter a scaling of inputs on the user interface to a speed of articulation of the distal portion.
17. The method according to paragraph 14, wherein receiving the input includes actuating an auxiliary button disposed on a portion of the endoscope to inhibit articulation of the distal portion of the endoscope in one plane of articulation of the two planes of articulation.
18. The method according to paragraph 14, wherein receiving the input includes receiving an input from a workstation operably coupled to the endoscope.
19. The method according to paragraph 14, wherein manipulating the endoscope includes rotating the endoscope in a first direction to rotate the distal portion of the endoscope to the desired direction.
20. The method according to paragraph 14, further comprising causing a drive mechanism to articulate the distal portion of the endoscope in the one plane of articulation in response to the input.

## Claims

1. An endoscope, comprising:
a proximal portion;
a distal portion selectively articulatable in two planes of articulation; and
a user interface disposed on a portion of the proximal portion, the user interface operably coupled to the distal portion such that inputs received on the user interface effectuate articulation of the distal portion,
wherein a portion of the user interface is actuatable to inhibit articulation of the distal portion in one plane of articulation of the two planes of articulation via inputs on the user interface.

2. The endoscope according to claim 1, wherein the user interface includes a joystick.

3. The endoscope according to claim 1 or claim 2, wherein the user interface includes a button pad.

4. The endoscope according to any preceding claim, wherein the user interface includes an auxiliary switch, the auxiliary switch selectively actuatable to enable or disable articulation of the distal portion in one of the two planes of articulation.

5. The endoscope according to any preceding claim, further comprising a drive mechanism disposed within a portion of the proximal portion, the drive mechanism operably coupled to the user interface and operably coupled to the distal portion such that inputs received by the user interface cause the drive mechanism to effectuate articulation of the distal portion.

6. The endoscope according to claim 5, wherein the user interface is actuatable to inhibit actuation of a portion of the drive mechanism, such that the drive mechanism is constrained to articulating the distal portion in one plane of articulation of the two planes of articulation.

7. The endoscope according to any preceding claim, wherein a portion of the user interface is actuatable to alter a scaling of inputs on the user interface to a speed of articulation of the distal portion.

8. A system for performing a surgical procedure, comprising:
an endoscope, the endoscope including:
a distal portion selectively articulatable in two planes of articulation; and
a user interface, the user interface operably coupled to the distal portion such that inputs received on the user interface effectuate articulation of the distal portion; and
a workstation operably coupled to the endoscope, the workstation including a memory and a processor, the memory storing instructions, which when executed by the processor cause the processor to:
receive an input; and
in response to the input, inhibit articulation of the distal portion of the endoscope in one plane of articulation of the two planes of articulation.

9. The system according to claim 8, wherein the instructions, when executed by the processor, altering a scaling of inputs on the user interface to a speed of articulation of the distal portion in response to the input.

10. The system according to claim 8 or claim 9, wherein the endoscope further includes a drive mechanism, the drive mechanism operably coupled to the user interface and operably coupled to the distal portion such that inputs received by the user interface cause the drive mechanism to effectuate articulation of the distal portion.

11. The system according to any of claims 8 to 10, wherein receiving an input includes receiving an input from an auxiliary switch operably coupled to the user interface.

12. The system according to any of claims 8 to 11, wherein receiving an input includes receiving an input from the instructions stored on the memory.

13. The system according to any of claims 8 to 12, wherein the user interface includes a joystick.
